# EUROPEAN PATENT SPECIFICATION

(11) **EP 0 329 397 B1**
(45) Date of publication and mention of the grant of the patent: **27.10.1993**
(21) Application number: 89301420.9
(22) Date of filing: 15.02.1989
(51) Int. Cl.: C07D 249/08, C07D 233/60

(54) **Process for producing azolylmethylcyclopentanol derivatives**
Verfahren zur Herstellung von Azolylmethyl-cyclopentanol-Derivaten
Procédé de préparation de dérivés de l'azolylméthylcyclopentanol

(30) Priority: 16.02.1988 JP 33773/88; 14.09.1988 JP 231338/88
(43) Date of publication of application: 23.08.1989
(73) Proprietor: KUREHA KAGAKU KOGYO KABUSHIKI KAISHA, Chuo-ku Tokyo 103 (JP)
(72) Inventor: Sunagawa, Kazuhiko, Iwaki-shi Fukushima-ken (JP); Hoshi, Hajime, Iwaki-shi Fukushima-ken (JP)
(74) Representative: Woods, Geoffrey Corlett

(56) References cited:
- EP-A- 0 267 778
- DE-A- 3 630 840

## Description

The present invention relates to a process for producing an azolylmethylcyclopentanol derivative represented by the formula (II): 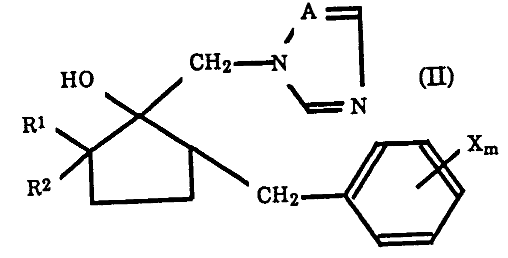
wherein R¹ represents a hydrogen atom or an alkyl group having 1 to 5 carbon atoms, R² represents a hydrogen atom or an alkyl group having 1 or 2 carbon atoms, X represents a halogen atom, an alkyl group having 1 to 4 carbon atoms, a phenyl group, a CN group, a CF₃ group or a NO₂ group, m is an integer of 1 to 5 and A represents a nitrogen atom or a CH group provided that when X is phenyl m is not 3,4 or 5. The invention also relates to a process for forming an agricultural or horticultural composition comprising the azolylmethylcyclopentanol derivative so prepared, to a process for controlling a fungal infection on plants or regulating the growth of plants and to a process for killing weeds at a locus.

Some methods of producing an azolylmethylcyclopentanol derivative from a cyclopentanone derivative have been known. For example, GB-A-2,180,236 discloses a method of reacting a cyclopentanone derivative with dimethyloxosulfonium methylide in a presence of a diluent, obtaining an oxirane derivative and thereafter reacting the oxirane derivative with an azole derivative. However, in case of producing an azolylmethylcyclopentanol derivative in two steps as is described above, the yield of the oxirane derivative in the first step is about 50 to 60% and the yield of the azole derivative in the second step is about 60 to 70%, accordingly the total yield being unsatisfactory. During the reaction for producing an oxirane derivative, which is usually carried out at a temperature of -10 to 80°C, a by-product such as an oxetane derivative is formed. If the reaction temperature is high, the formation of a by-product increases, while if the reaction temperature is lowered to avoid the formation of a by-product, the reaction hardly reaches its completion.

As described above, in the method of producing an azolylmethylcyclopentanol derivative represented by the formula (II) from a cyclopentanone derivative, there are several disadvantages, for example, (1) two-step reaction is necessary, (2) a large amount of by-product can not be avoided and (3) the yield cannot be sufficiently high.

The present inventors have developed a process for producing an azolylmethylcyclopentanol derivative, which is the target compound, with a high yield, suppressing the formation of an oxetane compound, a by-product, by performing the reaction for producing an oxirane derivative and the reaction for producing an azole derivative in one reaction vessel while using a specific solvent, preferably a specific solvent mixture and the present invention has been achieved by this development.

The present invention provides a process for producing an azolylmethylcyclopentanol derivative represented by the formula (II): 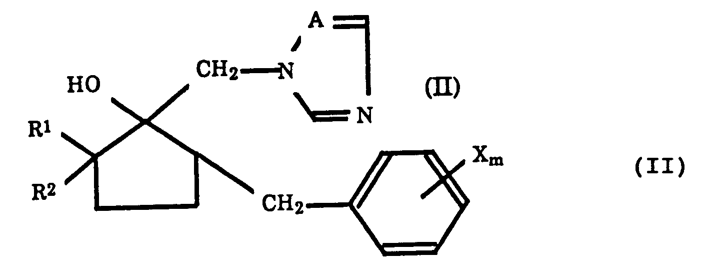
wherein R¹ represents a hydrogen atom or an alkyl group having 1 to 5 carbon atoms, R² represents a hydrogen atom or an alkyl group having 1 or 2 carbon atoms, X represents a halogen atom, an alkyl group having 1 to 4 carbon atoms, a phenyl group, a CN group, a CF₃ group or a NO₂ group, m is an integer of 1 to 5 and A represents a nitrogen atom or a CH group provided that when X is a phenyl group m is not 3, 4 or 5; which process comprises reacting a cyclopentanone derivative represented by the formula (I): 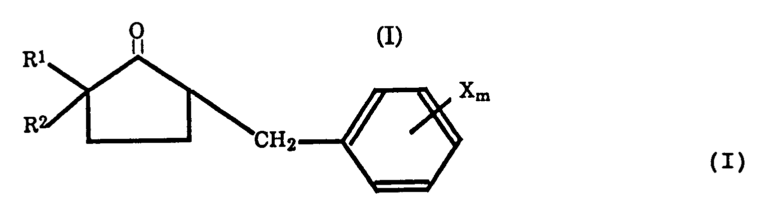
wherein R¹, R², X and m are the same as defined in the formula (II) with an azole compound represented by the formula (III): 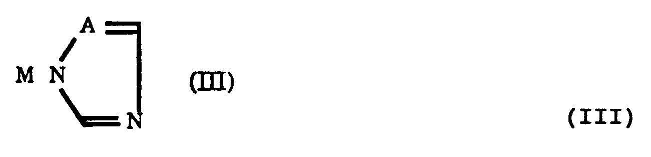
wherein A represents a nitrogen atom or a CH group and M represents an alkali metal atom or a hydrogen atom, and a sulfonium methylide or a sulfoxonium methylide in a polar solvent or a mixture of a polar solvent and an alcohol having 1 to 5 carbon atoms and, when M represents a hydrogen atom, in the presence of a base.

The present invention therefore provides a method of producing an azolylmethylcyclopentanol derivative represented by the above formula (II), the target compound, in a one-step reaction by reacting a cyclopentanone derivative represented by the formula (I) with an imidazole or 1,2,4-triazole represented by the formula (III) in a polar solution in the presence of a sulfonium methylide or a sulfoxonium methylide.

The polar solvent, preferably dimethyl sulfoxide or a polar solvent having an amide bond such as N-methyl-2-pyrrolidone, dimethylformamide or dimethylacetomamide can be used singly. However, such a polar solvent is preferably used together with an alcohol having 1 to 5 carbon atoms in the form of a mixture. A mixture of N-methyl-2-pyrrolidone and tert-butyl alcohol is particularly preferably. The mixing ratio of the polar solvent and the alcohol can be optional, but is preferably from 20:80 to 95:05 by volume, more preferably 50:50 to 95:05 by volume.

When M in the azole derivative represented by the formula (III) is a hydrogen atom, the reaction with a sulfonium methylide or a sulfoxonium methylide is carried out in the presence of a base and when M is an alkali metal atom, the presence of a base is not always necessary.

It is disclosed in GB-A-2,180,236 and EP-A-0,267,778 that azolylmethylcyclopentanol derivatives produced by a process according to the present invention are useful as agricultural and horticultural fungicides.

As the methylide, dimethylsulfoxonium methylide and dimethylsulfonium methylide are usable.

Examples of the base are a carbonate of an alkali metal such as sodium carbonate and potassium carbonate; a hydroxide of an alkali metal such as sodium hydroxide and potassium hydroxide; an alkoxide of an alkali metal such as sodium methoxide, sodium ethoxide, sodium tert-butoxide and potassium tert-butoxide; a hydride of an alkali metal such as sodium hydride and potassium hydride; an organic metal compound of an alkali metal such as n-butyl lithium; and others, such as, triethylamine and pyridine.

When a process according to the present invention is to be performed, a cyclopentanone derivative represented by the formula (I) is reacted with the imidazole or 1,2,4-triazole in the above solvent in the presence of a base and a methylide or an alkali metal salt of imidazole or of 1,2,4-triazole in the above solvent and a methylide. To explain this more concretely, a solution of a cyclopentanone derivative dissolved in the above solvent is reacted with a methylide prepared separately and a solution of an alkali metal salt of imidazole or of 1,2,4-triazole dissolved in the same solvent.

According to a preferred embodiment, the reaction is carried out by dissolving said cyclopentanone derivative and said azole compound in said polar solvent or said mixture and intermittently adding a trimethylsulfonium halide or a trimethylsulfoxonium halide and a base to the solution. According to another preferred embodiment, the reaction is carried out by dissolving said cyclopentanone derivative, said azole compound and said base in said polar solvent or said mixture and intermittently adding a trimethylsulfonium halide or a trimethylsulfoxonium halide to the solution.

When the methylide prepared in advance and separately is used, it is also preferable to add the methylide dividing it into 3 to 10 portions or dropwise during 3 to 7 hours.

The amount of dimethyloxosulfonium methylide or dimethylsulfonium methylide and the amount of azole to be added are preferably 1.0 to 2.0 equivalents of a cyclopentanone derivative used.

The reaction temperature can be any temperature ranging from the freezing point to the boiling point of the solvent used, but practically, it is preferable that the reaction is carried out at a temperature of room temperature to 120°C, more preferably 70 to 110°C. The reaction is carried out for 3 to 20 hours, 3 to 10 hours in many instances, preferably with stirring.

After the completion of the reaction, the reaction mixture obtained is cooled, and thereafter poured into iced water then extracted with an organic solvent such as ethyl acetate, chloroform, methylene chloride, benzene and toluene. The organic layer separated is then washed with water and dried and thereafter the solvent is distilled off under a reduced pressure. The residue is purified to obtain the target compound, namely, an azolylmethylcyclopentanol derivative.

In a process for producing an azolylmethylcyclopentanol derivative according to the present invention, the reacting operation is simpler than in a conventional method which requires a two-step reaction and it is possible to obtain the target compound with a higher yield only with a small amount of by-product.

The present invention also provides a process for forming an agricultural or horticultural composition which process comprises preparing an azolylmethylcyclopentanol derivative of formula (II) in accordance with the present invention and formulating the said azolylmethylcyclopentanol derivative thus prepared with a suitable carrier and/or diluent. The invention also provides a process for controlling a fungal infection on plants or regulating the growth of plants which process comprises preparing an azolylmethylcyclopentanol derivative of formula (II) in accordance with the present invention and applying to the plants an effective amount of the said azolylmethylcyclopentanol derivative. The invention yet further provides a process for killing weeds at a locus which process comprises preparing an azolylmethylcyclopentanol derivative of formula (II) in accordance with the present invention and applying to the locus a herbicidally effective amount of the said azolylmethylcyclopentanol derivative.

The following examples illustrate the process of the invention for the preparation of on azolylcyclopentanol derivative.

### Example 1:

Into a 300-ml four-necked flask equipped with a cooler having a calcium chloride cylinder, an agitator, a thermometer and a nitrogen-introducing pipe, 3.5 g (0.0875 mol) of 60% oily sodium hydride was charged, washed with dried hexane and hexane was removed. 30 ml of N-methyl-2-pyrrolidone was added thereto and the mixture was stirred. 6.49 g (0.0375 mol) of trimethylsulfoxonium bromide was added to the reaction mixture in a small quantity at a time and prepared dimethyloxosulfonium methylide.

After bubbling was over, 3.45 g (0.05 mol) of 1,2,4-triazole was added in a small quantity at a time. Alter stirring the whole mixture for 30 minutes, 30 ml of tert-amyl alcohol and 5.9 g (0.025 mol) of 5-(4-chlorobenzyl)-2,2-dimethylcyclopentanone were added and the mixture was stirred at 100°C for 4 hours.

After cooling the reaction solution to room temperature, it was poured into iced water, extracted with chloroform and obtained an organic layer. The layer was washed with water and dried over anhydrous sodium sulfate and chloroform was then distilled off to obtain 9.3 g of an oily material.

By purifying the oily material with column chromatography using silica gel, 4.37 g of the target compound, 5-(4-chlorobenzyl)-1-(1H-1,2,4-triazol-1-ylmethyl)-2,2-dimethylcyclopentanol was obtained.
(Yield: 54.7%, m.p. 114 to 116°C.)

### Examples 2 and 3:

The same target compound was synthesized in the same manner as in Example 1 except that 2-propyl alcohol and 2-butyl alcohol were respectively used in place of tert-amyl alcohol. The results are shown in Table 1.

**Table 1**

| | Example 2 | Example 3 |
|---|---|---|
| Alcohol Used | 2-Propyl alcohol | 2-Butyl alcohol |
| Yield % of the Target Compound | 50.2 | 53.2 |

### Example 4:

(1) Into a 2-l four-necked flask equipped with a cooler having a calcium chloride cylinder, an agitator, a thermometer and a nitrogen-introducing pipe, 32 g of 60% oily sodium hydride was charged, washed with dried hexane and hexane was removed. 200 ml of N-methyl-2-pyrrolidone was added thereto and the mixture was stirred. 55.2 g of 1, 2, 4-triazole was added to the reaction mixture and stirred at room temperature until bubbling was over. 200 ml of tert-butyl alcohol and 100 g of 2-(4-chlorobenzyl)-5-(2-propyl)cyclopentanone were then added and the mixture was heated. When the temperature reached 100°C, 120 ml of the solution of methylide prepared separately as follow was added and each 120 ml of the same solutions of methylide was further added after 1.5 and 3 hours respectively. The whole mixture was thereafter stirred at 100°C for 2 hours.
   After leaving the reaction solution to cool, it was poured into iced water, extracted with ethyl acetate and obtained an organic layer. The layer was washed with water and a saturated saline solution and dried over anhydrous sodium sulfate. Ethyl acetate was then distilled off to obtain 126.2 g of an oily material.
   By purifying the oily material with column chromatography using silica gel, 58.5 g of the target compound, 2-(4-chlorobenzyl)-1-(1H-1,2,4-triazol-1-ylmethyl)-5-(2-propyl)cyclopentanol was obtained in the form of white crystals. (Yield: 43.8%; m.p 75 to 81°C.)
(2) Preparation of a Solution of Methylide.
   Under a current of nitrogen, 24 g of 60% oily sodium hydride was washed with dried hexane; 50 ml of N-methyl-2-pyrrolidone was added thereto and 150 ml of tert-butyl alcohol was further added to the mixture. After bubbling was over, 132 g of trimethylsulfoxonium iodide was added and the mixture was stirred for 1 hour at 40°C, thereby obtaining the solution of methylide to be used in above (1).

### Example 5:

(1) Under a current of nitrogen, 480 mg of sodium hydride (60% oily sodium hydride washed with dried hexane) was added to 8 ml of N-methyl-2-pyrrolidone. 1.34 g of imidazole was then added thereto and the mixture was stirred at room temperature until bubbling was over. 8 ml of tert-butyl alcohol was then added and 2.37 g of 5-(4-chlorobenzyl)-2,2-dimethylcyclopentanone was added to the solution obtained. The mixture was heated. The solution of methylide, separately prepared as follow, was divided into three equal volume portions (each portion was about 3.3 ml) and one portion was added when the temperature reached 90°C, one portion after 2 hours and the last portion after 4 hours. The whole mixture was thereafter stirred at 90°C for 2 hours.
   After leaving the reaction solution to cool, it was poured into iced water, extracted with ethyl acetate and obtained an organic layer. The layer was washed with water and dried over anhydrous sodium sulfate and thereafter ethyl acetate was distilled off under a reduced pressure to obtain an oily material.
   By adding a small amount of n-hexane and ethyl acetate for solidification, 2.3 g of 5-(4-chlorobenzyl)-1-(1H-imidazol-l-ylmethyl)-2,2-dimethylcyclopentanol was obtained in the form of crystals. (Yield: 75%, m.p. 131 to 132°C.)
(2) Preparation of a Solution of Methylide.
   Under a current of nitrogen, 360 mg of sodium hydride (60% oily sodium hydride washed with dried hexane) was added to 5 ml of N-methyl-2-pyrrolidone. 5 ml of tert-butyl alcohol was added to the mixture. After bubbling was over, 3.3 g of trimethylsulfoxonium iodide was added and the mixture was stirred at 35 to 40°C for 1 hour, thereby obtaining the solution of methylide to be used in above (1).

### Example 6:

Under a current of nitrogen, 1.2 g of sodium hydride (60% oily sodium hydride washed with dried hexane) was added to 25 ml of N-methyl-2-pyrrolidone. 3.5 g of 1,2,4-triazole was then added thereto and the mixture was stirred at room temperature until bubbling was over. 5.9 g of 5-(4-chlorobenzyl)-2,2-dimethylcyclopentanone was added to the solution obtained. The mixture was heated and when the temperature reached 90°C, 7.5 ml of the solution of methylide, prepared separately in the same way as in Example 4, was added and the temperature was maintained at 100°C to promote the reaction. Each 7.5 ml of the solution of methylide was further added after 2 and 4 hours respectively and 2.5 ml of the solution was further added 2 hours thereafter and continued the reaction for another 2 hours.

After leaving the reaction solution to cool, it was poured into iced water, extracted with ethyl acetate and obtained an organic layer. The layer was washed with water and dried over anhydrous sodium sulfate and thereafter ethyl acetate was distilled off under a reduced pressure to obtain an oily material.

By purifying the oily material with column chromatography using silica gel, 5.63 g of the target compound, 5-(4-chlorobenzyl)-1-(1H-1,2,4-triazol-1-ylmethyl)-2,2-dimethylcyclopentanol was obtained.
(Yield: 70.5%).

### Example 7:

The same target compound was synthesized in the same manner as in Example 6 except that dimethylformamide was used in place of N-methyl-2-pyrrolidone. (Yield: 44.5%).

### Example 8:

The same target compound was synthesized in the same manner as in Example 6 except that dimethylacetoamide was used in place of N-methyl-2-pyrrolidone. (Yield: 48.2%).

### Example 9:

The same target compound was synthesized in the same manner as in Example 6 except that dimethylsulfoxide was used in place of N-methyl-2-pyrrolidone. (Yield: 46.2%).

### Example 10:

Under a current of nitrogen, 1.2 g of sodium hydride (60% oily sodium hydride washed with dried hexane) was added to N-methyl-2-pyrrolidone. 3.5 g of 1,2,4-triazole was then added thereto and the mixture was stirred at room temperature until bubbling was over. tert-butyl alcohol was then added to the mixture. The mixing ratio of the solvents was varied keeping the total amount of N-methyl-2-pyrrolidone and tert-butyl alcohol including those used for the preparation of the solution of methylide to be 60 ml. 5.9 g of 5-(4-chlorobenzyl)-2,2-dimethylcyclopentanone was added to the solution obtained. The mixture was heated. When the temperature reached 100°C, 7.5 ml of the solution of methylide prepared separately in the same way as in Example 4 was added. Each 7.5 ml of the solution was further added after 2 and 4 hours respectively and the reaction was continued for another 2 hours.

After leaving the reaction solution to cool, it was poured into iced water, extracted with chloroform and obtained an organic layer. The layer was washed with water and dried over anhydrous sodium sulfate and thereafter chloroform was distilled off to obtain an oily material.

By purifying the oily material with column chromatography using silica gel, the target compound, 5-(4-chlorobenzyl)-1-(1H-1,2,4-triazol-1-ylmethyl)-2,2-dimethylcyclopentanol was obtained. The results are shown in Table 2.

**Table 2**

| Amount of Solvent Used (ml) | | Yield of the Target Compound(%) |
|---|---|---|
| N-Methyl-2-pyrrolidone | tert-Butyl Alcohol | |
| 60 | 0 | 47.0 |
| 50 | 10 | 55.8 |
| 40 | 20 | 60.2 |
| 30 | 30 | 62.1 |
| 20 | 40 | 53.6 |

### Example 11:

(1) Under a current of nitrogen, 480 mg of sodium hydride (60% oily sodium hydride washed with dried hexane) was added to 8 ml of N-methyl-2-pyrrolidone. 1.38 g of 1,2,4-triazole was then added thereto and the mixture was stirred at room temperature until bubbling was over. 8 ml of tert-butyl alcohol and 2.08 g of 2-(4-chlorobenzyl)cyclopentanone were then added. To the mixture, the solution of sulfonium methylide prepared separately as follow was added and the whole mixture was stirred for 2 hours at room temperature and then at 80°C for another 1 hour. The reaction solution obtained was poured into iced water, thereby stopping the reaction. An organic layer obtained by extraction with ethyl acetate was washed with water and dried over anhydrous sodium sulfate and thereafter ethyl acetate was distilled off under a reduced pressure to obtain an oily material.
   By purifying the oily material with column chromatography using silica gel, 1.3 g of the target compound, 2-(4-chlorobenzyl)-1-(1H-1,2,4-triazol-1-ylmethyl)cyclopentanol was obtained in the form of a glassy solid.
   (Yield: 45%).
(2) Preparation of the solution of sulfonium methylide.
   Under a current of nitrogen, 350 mg of sodium hydride (60% oily sodium hydride washed with dried hexane) was added to 5 ml of N-methyl-2-pyrrolidone. 5 ml of tert-butyl alcohol was added to the mixture. After bubbling was over, 3.06 g of trimethylsulfonium iodide was added in a small quantity at a time and the mixture was stirred for 30 minutes at room temperature, thereby obtaining the solution of sulfonium methylide to be used in above (1).

### Example 12:

Under a current of nitrogen, 6 g of sodium hydride (60% oily sodium hydride washed with dried hexane) was added to 100 ml of N-methyl-2-pyrrolidone. 11.1 g of tert-butyl alcohol was then added dropwise thereto and the mixture was stirred at 30°C until bubbling was over. 10.3 g of 1,2,4-triazole was added to the solution and the obtained solution was heated. When the temperature of the solution reached 100°C, 23.7 g of 5-(4-chlorobenzyl)-2,2-dimethylcyclopentanone was added. To the obtained solution, sodium tert-butoxide solution prepared separately as follow and trimethylsulfonium bromide were added in accordance with the following schedule to promote the reaction.

| Schedule | Sodium tert-Butoxide Solution Added (ml) | Trimethylsulfoxonium bromide Added (g) |
|---|---|---|
| Start of Reaction | 15 | 5.2 |
| After 1 hour | 15 | 5.2 |
| After 2 hours | 15 | 5.2 |
| After 3 hours | 5 | 5.2 |
| After 4 hours | -- | 2.6 |
| After 5 hours | -- | 2.6 |
| After 8 hours, the reaction is completed. | | |

After pouring the reaction solution obtained into iced water, it was extracted with ethyl acetate and obtained an organic layer. The layer was washed with water and dried over anhydrous sodium sulfate and ethyl acetate was distilled off under a reduced pressure to obtain an oily material.

By purifying the oily material with column chromatography using silica gel, 23.1 g of the target compound, 5-(4-chlorobenzyl)-1-(1H-1,2,4-triazol-1-ylmethyl)-2,2-dimethylcyclopentanol was obtained. (Yield: 72.2%).

The sodium tert-butoxide solution was prepared by adding 2.4 g of sodium hydride (60% oily sodium hydride washed with dried hexane) to 40 ml of N-methyl-2-pyrrolidone under a current of nitrogen, thereafter adding dropwise 7.4 g of tert-butyl alcohol and stirring the mixture at 30°C until bubbling was over.

### Example 13:

Into a 300-ml four-necked flask equipped with a cooler having a calcium chloride cylinder, an agitator, a thermometer and a nitrogen-introducing pipe, 3.5 g (0.0875 mol) of 60% oily sodium hydride was charged, washed with dried hexane and hexane was removed. 30 ml of N-methyl-2-pyrrolidone was added thereto and the mixture was stirred. 6.49 g (0.0375 mol) of trimethylsulfoxonium bromide was added to the reaction mixture in a small quantity at a time to prepare dimethyloxosulfonium methylide.

After bubbling was over, 3.40 g (0.05 mol) of imidazole was added in a small quantity at a time and the mixture was stirred for 30 minutes. Thereafter, 30 ml of tert-amyl alcohol and 4.9 g (0.025 mol) of 2-(4-cyanobenzyl)cyclopentanone were added and the mixture was stirred at 100°C for 4 hours.

After cooling the reaction solution to room temperature, it was poured into iced water, extracted with chloroform and obtained an organic layer. The layer was washed with water and dried over anhydrous sodium sulfate and chloroform was then distilled off to obtain an oily material.

By purifying the oily material with column chromatography using silica gel, 3.28 g of the target compound, 2-(4-cyanobenzyl)-1-(imidazol-1-ylmethyl)cyclopentanol was obtained. (Yield: 53.8%, m.p. 103 to 104°C).

### Example 14:

Into a 300-ml four-necked flask equipped with a cooler having a calcium chloride cylinder, an agitator, a thermometer and a nitrogen-introducing pipe, 3.5 g (0.0875 mol) of 60% oily sodium hydride was charged, washed with dried hexane and hexane was removed. 30 ml of N-methyl-2-pyrrolidone was added thereto and the mixture was stirred. 6.49 g (0.0375 mol) of trimethylsulfoxonium bromide was added to the reaction mixture in a small quantity at a time to prepare dimethyloxosulfonium methylide.

After bubbling was over, 3.45 g (0.05 mol) of 1,2,4-triazole was added in a small quantity at a time. After stirring the mixture for 30 minutes, 30 ml of tert-amyl alcohol and 6.0 g (0.025 mol) of 2-(3-trifluoromethylbenzyl)cyclopentanone were added and the mixture was stirred at 100°C for 4 hours.

After cooling the reaction solution to room temperature, it was poured into iced water, extracted with chloroform and obtained an organic layer. The layer was washed with water and dried over anhydrous sodium sulfate and chloroform was distilled off to obtain an oily material.

By purifying the oily material with column chromatography using silica gel, 3.87 g of the target compound, 2-(3-trifluoromethylbenzyl)-1-(1H-1,2,4-triazol-1-ylmethyl)cyclopentanol was obtained.
(Yield: 53.6%, m.p. 152 to 153°C).

### Example 15:

Into a 300-ml four-necked flask equipped with a cooler having a calcium chloride cylinder, an agitator, a thermometer and a nitrogen-introducing pipe, 3.5 g (0.0875 mol) of 60% oily sodium hydride was charged, washed with dried hexane and hexane was removed. 30 ml of N-methyl-2-pyrrolidone was added thereto and the mixture was stirred. 6.49 g (0.0375 mol) of trimethylsulfoxonium bromide was added to the reaction mixture in a small quantity at a time to prepare dimethyloxosulfonium methylide.

After bubbling was over, 3.45 g (0.05 mol) of 1,2,4-triazole was added in a small quantity at a time. After stirring the mixture for 30 minutes, 30 ml of tert-amyl alcohol and 5.4 g (0.025 mol) of 2-(4-nitrobenzyl)cyclopentanone were added and the mixture was stirred at 100°C for 4 hours.

After cooling the reaction solution to room temperature, it was poured into iced water, extracted with chloroform and obtained an organic layer. The layer was washed with water and dried over anhydrous sodium sulfate and chloroform was then distilled off to obtain an oily material.

By purifying the oily material with column chromatography using silica gel, 3.60 g of the target compound, 2-(4-nitrobenzyl)-1-(1H-1,2,4-triazol-1-ylmethyl)cyclopentanol was obtained. (Yield: 54.2%, m.p. 131 to 132°C).

### Example 16:

Into a 300-ml four-necked flask equipped with a cooler having a calcium chloride cylinder, an agitator, a thermometer and a nitrogen-introducing pipe, 3.5 g (0.0875 mol) of 60% oily sodium hydride was charged, washed with dried hexane and hexane was removed. 30 ml of N-methyl-2-pyrrolidone was added thereto and the mixture was stirred. 6.49 g (0.0375 mol) of trimethylsulfoxonium bromide was added to the reaction mixture in a small quantity at a time to prepare dimethyloxosulfonium methylide.

After bubbling was over, 3.45 g (0.05 mol) of 1,2,4-triazole was added in a small quantity at a time. After stirring the mixture for 30 minutes, 30 ml of tert-amyl alcohol and 6.3 g (0.025 mol) of 2-(4-phenylbenzyl)cyclopentanone were added and the mixture was stirred at 100°C for 4 hours.

After cooling the reaction solution to room temperature, it was poured into iced water, extracted with chloroform and obtained an organic layer. The layer was washed with water and dried over anhydrous sodium sulfate and chloroform was then distilled off to obtain an oily material.

By purifying the oily material with column chromatography using silica gel, 4.10 g of the target compound, 2-(4-phenylbenzyl)-1-(1H-1,2,4-triazol-1-ylmethyl)cyclopentanol was obtained. (Yield: 50.8%, m.p. 146 to 147°C).

### Example 17:

Under a current of nitrogen, 3.6 g of sodium hydride (60% oily sodium hydride washed with dried hexane) was added to 100 ml of N-methyl-2-pyrrolidone. 11.1 g of tert-butyl alcohol was then added thereto and the mixture was stirred at room temperature until bubbling was over. 10.35 g of 1,2,4-triazole and 23.6 g of 5-(4-chlorobenzyl)-2,2-dimethylcyclopentanone were then added to the mixture and the whole mixture was heated. When the temperature reached 105°C, 9.5 g of sodium tert-butoxide, which had been prepared separately in 40 ml of N-methyl-2-pyrrolidone (cf. Example 12), and 5.2 g of trimethylsulfoxonium bromide were added and the temperature was maintained at 105°C. Each 5.2 g of trimethylsulfoxonium bromide was added after 30 minutes, 1 hour and 1.5 hours respectively and each 2.6 g of trimethylsulfoxonium bromide was subsequently added after 2 hours and 2.5 hours, also respectively, and continued the reaction for another 1.5 hours.

After leaving the reaction solution to cool, it was poured into iced water and extracted with toluene to obtain an organic layer. The organic layer was washed with water and dried over anhydrous sodium sulfate, and thereafter toluene was distilled off to obtain an oily material.

By purifying the oily material with column chromatography using silica gel, 20.1 g of the target compound, 5-(4-chlorobenzyl)-1-(1H-1,2,4-triazol-1-ylmethyl)-2,2-dimethylcyclopentanol was obtained. (Yield: 62.8%).

## Claims

1. A process for producing an azolylmethylcyclopentanol derivative represented by the formula (II): 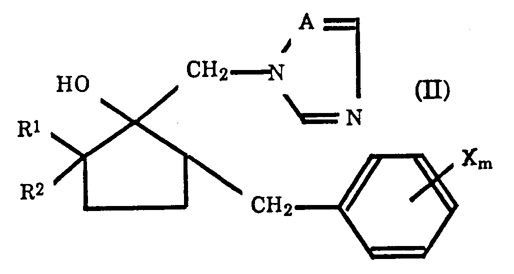 where R¹ represents a hydrogen atom or an alkyl group having 1 to 5 carbon atoms, R² represents a hydrogen atom or an alkyl group having 1 or 2 carbon atoms, X represents a halogen atom, an alkyl group having 1 to 4 carbon atoms, a phenyl group, a cyano group, a trifluoromethyl group or a nitro group, m is an integer of 1 to 5 and A represents a nitrogen atom or a CH group provided that when X is phenyl m is not 3, 4 or 5; which process comprises reacting a cyclopentanone derivative represented by the formula (I): 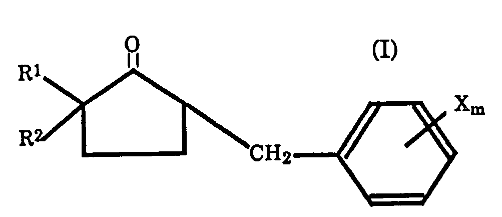 wherein R¹, R², X and m are as defined above, with an azole compound represented by the formula (III): 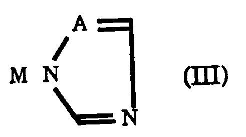 wherein A is as defined above and M represents an alkali metal atom or a hydrogen atom, and a sulfonium methylide or a sulfoxonium methylide in a polar solvent or a mixture of a polar solvent and an alcohol having 1 to 5 carbon atoms and, when M represents a hydrogen atom, in the presence of a base.

2. A process according to claim 1, wherein the reaction is carried out by dissolving said cyclopentanone derivative and said azole compound in said polar solvent or said mixture and intermittently adding a trimethylsulfonium halide or a trimethylsulfoxonium halide and a base to the solution.

3. A process according to claim 1, wherein the reaction is carried out by dissolving said cyclopentanone derivative, said azole compound and said base in said polar solvent or said mixture and intermittently adding a trimethylsulfonium halide or a trimethylsulfoxonium halide to the solution.

4. A process according to any one of the preceding claims, wherein said polar solvent is dimethylsulfoxide or a polar solvent having an amide bond.

5. A process according to claim 4, wherein said polar solvent having an amide bond is selected from N-methyl-2-pyrrolidone, dimethylformamide and dimethylacetamide.

6. A process according to any one of the preceding claims, wherein the mixing ratio of said polar solvent and said alcohol is from 20:80 to 95:05 by volume.

7. A process according to any one of the preceding claims, wherein said mixture is a mixture of N-methyl-2-pyrrolidone and tert-butyl alcohol.

8. A process for forming an agricultural or horticultural composition which process comprises preparing an azolymethylcyclopentanol derivative of formula (II) by the process of any one of claims 1 to 7 and formulating the said azolylmethylcyclopentanol derivative thus prepared with a suitable carrier and/or diluent.

9. A process for controlling a fungal infection on plants or regulating the growth of plants which process comprises preparing an azolylmethylcyclopentanol derivative of formula (II) by the process of any one of claims 1 to 7 and applying to the plants an effective amount of the said azolylmethylcyclopentanol derivative thus obtained.

10. A process for killing weeds at a locus which process comprises preparing an azolylmethylcyclopentanol derivative of formula (II) by the process of any one of claims 1 to 7 and applying to the locus a herbicidally effective amount of the said azolylmethylcyclopentanol derivative thus obtained.

## Patentansprüche

1. Verfahren zur Herstellung eines Azolylmethylcyclopentanolderivats, dargestellt durch die Formel (II): 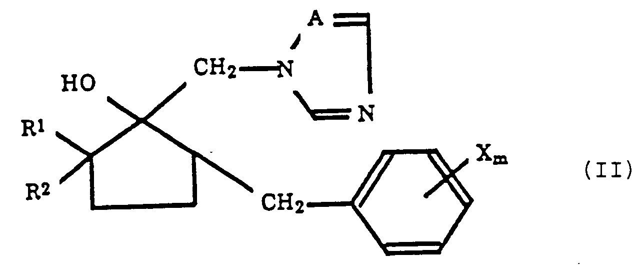 worin R¹ ein Wasserstoffatom oder eine Alkylgruppe mit 1 bis 5 Kohlenstoffatomen darstellt, R² ein Wasserstoffatom oder eine Alkylgruppe mit 1 oder 2 Kohlenstoffatomen darstellt, X ein Halogenatom, eine Alkylgruppe mit 1 bis 4 Kohlenstoffatomen, eine Phenylgruppe, eine Cyanogruppe, eine Trifluormethylgruppe oder eine Nitrogruppe darstellt, m eine ganze Zahl von 1 bis 5 ist und A ein Stickstoffatom oder eine CH-Gruppe darstellt, unter der Voraussetzung, dass, wenn X eine Phenylgruppe ist, m nicht 3, 4 oder 5 ist; wobei das Verfahren umfasst:
Umsetzung eines Cyclopentanonderivats, dargestellt durch die Formel (I): 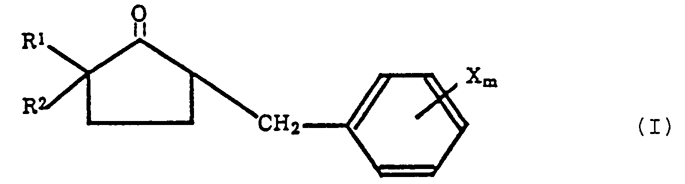 worin R¹, R², X und m wie oben definiert sind, mit einer Azolverbindung, dargestellt durch die Formel (III): 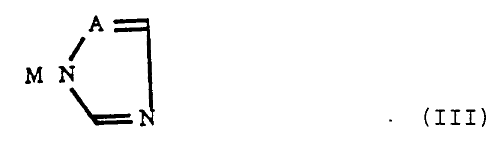 worin A wie oben definiert ist und M ein Alkalimetallatom oder ein Wasserstoffatom darstellt, und einem Sulfoniummethylid oder einem Sulfoxoniummethylid in einem polaren Lösungsmittel oder einer Mischung eines polaren Lösungsmittels und eines Alkohols mit 1 bis 5 Kohlenstoffatomen und, wenn M ein Wasserstoffatom darstellt, in Gegenwart einer Base.

2. Verfahren gemäss Anspruch 1, dadurch **gekennzeichnet,** dass die Reaktion durch Auflösung des Cyclopentanonderivats und der Azolverbindung in dem polaren Lösungsmittel oder der Mischung und durch portionsweise Zugabe eines Trimethylsulfoniumhalogenids oder eines Trimethylsulfoxoniumhalogenids und einer Base zu der Lösung ausgeführt wird.

3. Verfahren gemäss Anspruch 1, dadurch **gekennzeichnet,** dass die Reaktion durch Auflösung des Cyclopentanonderivats, der Azolverbindung und der Base in dem polaren Lösungsmittel oder der Mischung und durch portionsweise Zugabe eines Trimethylsulfoniumhalogenids oder eines Trimethylsulfoxoniumhalogenids zu der Lösung ausgeführt wird.

4. Verfahren gemäss einem der vorhergehenden Ansprüche, dadurch **gekennzeichnet,** dass das polare Lösungsmittel Dimethylsulfoxid oder ein polares Lösungsmittel mit einer Amidbindung ist.

5. Verfahren gemäss Anspruch 4, dadurch **gekennzeichnet,** dass das polare Lösungsmittel mit einer Amidbindung ausgewählt wird aus N-Methyl-2-pyrrolidon, Dimethylformamid und Dimethylacetamid.

6. Verfahren gemäss einem der vorhergehenden Ansprüche, dadurch **gekennzeichnet,** dass das Mischungsverhältnis des polaren Lösungsmittels und des Alkohols von 20:80 bis 95:5 Volumenteile beträgt.

7. Verfahren gemäss einem der vorhergehenden Ansprüche, dadurch **gekennzeichnet,** dass die Mischung eine Mischung aus N-Methyl-2-pyrrolidon und tert-Butylalkohol ist.

8. Verfahren zur Herstellung einer Zusammensetzung für Landwirtschaft oder Gartenbau, umfassend die Herstellung eines Azolylmethylcyclopentanolderivats der Formel (II) nach dem Verfahren gemäss einem der Ansprüche 1 bis 7, und Formulierung dieses so hergestellten Azolylmethylcyclopentanolderivats mit einem geeigneten Träger und/oder Verdünnungsmittel.

9. Verfahren zur Kontrolle einer Pilzinfektion auf Pflanzen oder zur Regulierung des Pflanzenwachstums, umfassend die Herstellung eines Azolylmethylcyclopentanolderivats der Formel (II) nach dem Verfahren gemäss einem der Ansprüche 1 bis 7, und Aufbringen einer wirksamen Menge dieses so erhaltenen Azolylmethylcyclopentanolderivats auf die Pflanzen.

10. Verfahren zur Vernichtung von Unkräutern auf einer Stelle, wobei das Verfahren die Herstellung eines Azolylmethylcyclopentanolderivats der Formel (II) nach dem Verfahren gemäss einem der Ansprüche 1 bis 7 und Aufbringen einer herbizid wirksamen Menge dieses so erhaltenen Azolylmethylcyclopentanolderivats auf die Stelle umfasst.

## Revendications

1. Procédé de préparation d'un dérivé d'azolylméthylcyclopentanol représenté par la formule (II): 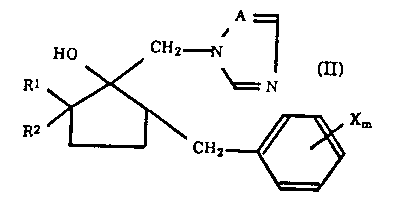 dans laquelle R¹ représente un atome d'hydrogène ou un groupe alkyle ayant de 1 à 5 atomes de carbone, R² représente un atome d'hydrogène ou un groupe alkyle ayant de 1 à 2 atomes de carbone, X représente un atome d'halogène, un groupe alkyle ayant de 1 à 4 atomes de carbone, un groupe phényle, un groupe cyano, un groupe trifluorométhyle ou un groupe nitro, m est un entier de 1 à 5 et A représente un atome d'azote ou un groupe CH pourvu que quand X représente phényle m ne soit pas 3, 4 ou 5; lequel procédé comprend la mise en réaction d'un dérivé de cyclopentanone représenté par la formule (I): 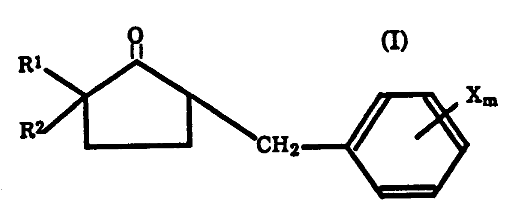 dans laquelle R¹, R², X et m sont définis ci-dessus, avec un composé azole représenté par la formule (III): 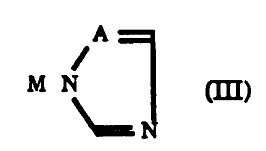 dans laquelle A est tel que défini ci-dessus et M représente un atome de métal alcalin ou un atome d'hydrogène, et un méthylure de sulfonium ou un méthylure de sulfoxonium dans un solvant polaire ou un mélange d'un solvant polaire et d'un alcool ayant de 1 à 5 atomes de carbone et, quand M représente un atome d'hydrogène, en présence d'une base.

2. Procédé selon la revendication 1, dans lequel la réaction est conduite en dissolvant ledit dérivé de cyclopentanone et ledit composé azole dans ledit solvant polaire ou ledit mélange et en ajoutant de façon intermittente un halogénure de triméthylsulfonium ou un halogénure de triméthylsulfoxonium et une base à la solution.

3. Procédé selon la revendication 1, dans lequel la réaction est conduite en dissolvant ledit dérivé de cyclopentanone, ledit composé azole et ladite base dans ledit solvant polaire ou ledit mélange et en ajoutant de façon intermittente un halogénure de triméthylsulfonium ou un halogénure de triméthylsulfoxonium à la solution.

4. Procédé selon l'une quelconque des revendications précédentes, dans lequel ledit solvant polaire est le diméthylsulfoxyde ou un solvant polaire ayant une liaison amide.

5. Procédé selon la revendication 4, dans lequel ledit solvant polaire ayant une liaison amide est choisi parmi N-méthyl-2-pyrrolidone, diméthylformamide et diméthylacétamide.

6. Procédé selon l'une quelconque des revendications précédentes, dans lequel le rapport du mélange dudit solvant polaire et dudit alcool est compris entre 20:80 et 95:05 en volume.

7. Procédé selon l'une quelconque des revendications précédentes, dans lequel ledit mélange est un mélange de N-méthyl-2-pyrrolidone et d'alcool t-butylique.

8. Procédé pour la formation d'une composition agricole ou horticole lequel procédé comprend une préparation d'un dérivé d'azolylméthylcyclopentanol de formule (II) par le procédé de n'importe laquelle des revendications 1 à 7 et la formulation dudit dérivé d'azolylméthylcyclopentanol ainsi préparé avec un support appropriée et/ou un diluant.

9. Procédé pour le contrôle d'une infection fongique de plantes ou de régulation de croissance de plantes lequel procédé comprend la préparation d'un dérivé d'azolylméthylcyclopentanol de formule (II) par le procédé de l'une quelconque des revendications 1 à 7 et l'application à des plantes en quantité efficace dudit dérivé d'azolylméthylcyclopentanol ainsi obtenu.

10. Procédé pour détruire les mauvaises herbes sur un site lequel comprend la préparation d'un dérivé d'azolylméthylcyclopentanol de formule (II) par le procédé de l'une quelconque des revendications de 1 à 7 et l'application sur le site d'une quantité efficace herbicide dudit dérivé d'azolyméthylcyclopentanol ainsi obtenu.
